(19) **Europäisches Patentamt**

**European Patent Office**

**Office européen des brevets**

(11) **EP 0 657 187 B1**

(12) **FASCICULE DE BREVET EUROPEEN**

(45) Date de publication et mention
de la délivrance du brevet:
**05.07.2000 Bulletin 2000/27**

(51) Int. Cl.⁷: **A61N 1/365**

(21) Numéro de dépôt: **94402775.4**

(22) Date de dépôt: **05.12.1994**

(54) **Stimulateur cardiaque implantable à fréquence asservie**

Implantierbarer Herzschrittmacher mit gesteuerter Frequenz

Implantable cardiac pacemaker with controlled frequency

(84) Etats contractants désignés:
**BE CH DE ES FR GB IT LI SE**

(30) Priorité: **06.12.1993 FR 9314578**

(43) Date de publication de la demande:
**14.06.1995 Bulletin 1995/24**

(73) Titulaire: **ELA MEDICAL**
**F-92541 Montrouge (FR)**

(72) Inventeurs:
• **Bouhour, Anne**
**F-75013 Paris (FR)**

• **Legay, Thierry**
**F-91640 Fontenay Les Briis (FR)**

(74) Mandataire: **Laget, Jean-Loup**
**Cabinet Loyer,**
**78, avenue Raymond Poincaré**
**75116 Paris (FR)**

(56) Documents cités:
**EP-A- 0 493 220        EP-A- 0 493 222**
**EP-A- 0 498 533        US-A- 5 074 302**

EP 0 657 187 B1

**Description**

L'invention s'applique à un stimulateur cardiaque implantable. Plus spécifiquement cette invention s'applique à un stimulateur cardiaque implantable à fréquence asservie dans lequel les caractéristiques de la stimulation délivrée peuvent évoluer en fonction d'un ou plusieurs paramètres liés à l'activité physique du porteur.

Il existe des stimulateurs cardiaques faisant évoluer le rythme de stimulation délivré en fonction d'un ou plusieurs paramètres liés à l'activité physique du porteur. Ces paramètres peuvent être par exemple la ventilation-minute, la température du sang, la concentration en oxygène du sang, le PH du sang, les vibrations recueillies sur le boîtier du stimulateur, les accélérations basse fréquence du tronc du porteur, l'intervalle QT, ou d'autres paramètres mesurables.

Parmi tous les paramètres mesurés par les capteurs adéquats, dits paramètres ou paramètres d'asservissement, certains se révèlent avoir une réaction un peu lente par rapport à celle du rythme cardiaque physiologique, c'est-à-dire naturel (par exemple la température du sang), ou trop rapide par rapport à celle-ci (par exemple les accélérations du tronc).

La relation entre le paramètre et la fréquence cardiaque correspondante (fréquence de consigne qui doit être atteinte) a donné lieu à plusieurs descriptions. Le brevet US 5,074,302 développe l'utilisation d'une relation linéaire ou non en fonction du paramètre, Le stimulateur comportant une variation de la fréquence suivant un schéma à trois catégories en cas de variation brusque de l'activité du patient. Le document WO 92/03183 évoque des relations associées à un ou plusieurs paramètres. Dans ces descriptions l'évolution temporelle de la fréquence stimulée n'est pas précisée dans le cas où la variation du paramètre est plus rapide que celle du rythme cardiaque physiologique. Il est donc nécessaire de définir temporellement comment la fréquence de stimulation cardiaque va évoluer de sa valeur courante à la valeur de consigne calculée, à partir du paramètre et d'une relation donnée.

Un but de l'invention est de proposer un stimulateur à fréquence asservie, en fonction d'un paramètre dont la variation est plus rapide que la réponse physiologique du rythme cardiaque, dans lequel une relation détermine l'évolution de la fréquence de stimulation.

Un autre but de l'invention est de proposer un stimulateur implanté à fréquence asservie à un paramètre dont la variation est plus rapide que celle du rythme cardiaque physiologique, dans lequel le profil d'évolution de la fréquence de stimulation est contrôlé.

L'invention a pour objet un stimulateur cardiaque implantable à fréquence asservie à au moins un paramètre représentatif de l'activité physique du porteur, dans lequel l'évolution de la fréquence de stimulation suit, de façon à reproduire l'évolution du rythme cardiaque physiologique, une fonction mathématique Fc(t), caractérisé en ce que :

$$Fc(t) = A * (1 - B * exp(-t/Tau)) + C,$$

où

Fc(t) est la fréquence cardiaque délivrée par le stimulateur au cours du temps,
t le paramètre de temps et A, B, C, Tau des constantes.

Selon d'autres caractéristiques :

Ladite fonction mathématique est approchée par un développement limité ;

La fonction mathématique est :

$$Fc(t) = K - (Tau/t) ;$$

Ladite fonction mathématique est du type :

- Fc(t) = (Fdépart - Fbut) exp(-t/Tau_mont) + Fbut , pour contrôler la croissance du rythme stimulé,
- Fc(t) = (Fdépart - Fbut) exp(-t/Tau_desc) + Fbut , pour contrôler la décroissance du rythme stimulé, où
- Fdépart est la fréquence cardiaque à partir de laquelle se produit l'accélération ou la décélération du rythme stimulé,
- Fbut est la fréquence cardiaque de consigne qui doit être atteinte et qui est calculée à partir de la valeur du ou des paramètres d'asservissement dudit stimulateur,
- Tau_mont est un paramètre de réglage de la croissance du rythme stimulé depuis Fdépart jusqu'à Fbut,
- Tau_desc est un paramètre de réglage de la décroissance du rythme stimulé depuis Fdépart jusqu'à Fbut ;

Ladite fonction mathématique est transformée pour s'appliquer à des intervalles d'échappement cardiaque et autoriser le calcul de l'intervalle d'échappement suivant en ajoutant à l'intervalle d'échappement courant la variation :

- dTc(t)=Tc$^3$(t)*[Fc(t)-Fbut]/Tau , où
- Tc(t)=1/Fc(t)
- Tau=Tau_mont, pour la croissance du rythme,
- Tau=Tau_desc, pour la décroissance du rythme ;

La variation dTc(t) n'est pas systématiquement ajoutée à chaque intervalle d'échappement mais à un intervalle d'échappement sur un nombre variable (MULT) d'intervalles d'échappement ;

Pour appliquer ladite variation dTc(t) à un intervalle d'échappement sur un nombre variable (MULT) d'intervalles d'échappement, ladite variation est calculée de la façon suivante :

- dTc(t)=Arrondi(MULT*Tc$^3$(t)*[Fc(t)-Fbut]/Tau) , où

le facteur MULT est obtenu par un algorithme ;

Le facteur MULT :

- est incrémenté quand ladite variation appliquée à l'intervalle d'échappement précédent est nulle,
- est remis à 1 dès que ladite variation est non nulle,
- est remis à 1 si Fc=Fbut ;

La variation autorisée de la fréquence cardiaque d'un cycle au suivant est limitée à une valeur maximale, de préférence égale à 6min$^{-1}$.

Sur le dessin annexé :

- La Figure 1a représente l'évolution d'un paramètre représentatif de l'activité d'un sujet au cours de diverses phases de repos, d'activité moyenne (marche), et d'activité intense (course) ;
- La Figure 1b représente l'évolution de la fréquence de stimulation cardiaque déterminée en application de l'invention, et correspondant aux mêmes périodes de repos, d'activité moyenne, et d'activité intense, que dans le cas de la Figure 1a ;
- La figure 1c représente l'évolution de la fréquence cardiaque naturelle d'un sujet sans pathologie cardiaque, mesurée par un électrocardiogramme externe, et correspondant aux mêmes périodes de repos, d'activité physique et d'activité intense que dans le cas de la Figure 1a ;
- La Figure 2 représente un organigramme de contrôle du profil de la fréquence de stimulation cardiaque, applicable à la détermination de la fréquence de stimulation de la Figure 1b.

L'invention s'applique à un stimulateur cardiaque à fréquence asservie à l'information d'un ou de plusieurs capteurs, représentative de l'activité physique du porteur. Le stimulateur est simple ou double chambre et possède toutes les fonctionnalités courantes d'un stimulateur cardiaque à fréquence asservie.

L'étude clinique ayant conduit à l'invention a montré que lors des efforts de la vie quotidienne (marche, marche rapide, course, etc), les sujets doivent fournir un niveau de puissance constant pendant l'effort, dont l'amplitude correspond au type d'effort effectué. D'autre part, pour chacun de ces efforts, la fréquence cardiaque croît suivant un profil bien défini jusqu'à atteindre un niveau représentatif de la puissance de l'effort.

L'invention est particulièrement efficace pour contrôler le profil d'évolution du rythme cardiaque d'un stimulateur à fréquence asservie à un paramètre dont la réaction est plus rapide que l'évolution physiologique du rythme cardiaque.

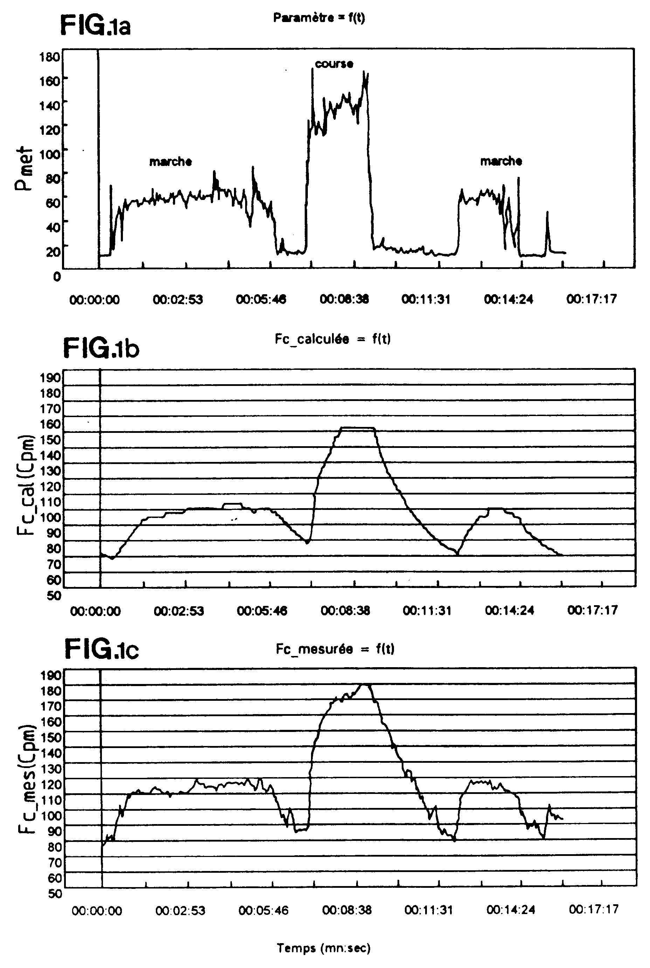

Les Figures 1a et 1c montrent un exemple d'évolution comparée du paramètre d'activité physique et de la fréquence cardiaque.

Le but de l'invention est de fournir une stimulation cardiaque dont le profil d'évolution à l'accélération ou à la décélération (Figure 1b) soit similaire au profil d'évolution physiologique (Figure 1c).

Le signal brut délivré par le(s) capteur(s) est traité par une chaîne électronique et/ou un logiciel pour fournir le(s) paramètre(s) d'asservissement. La valeur du ou des paramètres d'asservissement permet de calculer la valeur de la fréquence (Fcapt) qui est la fréquence cardiaque qui doit être atteinte après le régime transitoire. La fonction de transfert, utilisée dans l'application décrite, entre la valeur du paramètre et celle de la fréquence cardiaque correspondante, est une relation linéaire du type :

FCAPT=a*paramètre+b , où a et b sont programmés par le médecin et/ou déterminés de façon automatique en fonction de l'activité quotidienne du porteur. Cette relation entre Fcapt et le paramètre pourrait être tout autre. Le paramètre utilisé dans l'application est l'intégrale calculée sur 1,56s de la valeur absolue du signal d'accélération mesuré dans l'axe antéro-postérieur par un accéléromètre à variation de capacité. Ce paramètre d'asservissement fournit un niveau représentatif du niveau de puissance développé par le sujet pour l'effort donné et a une réponse plus rapide que la réponse cardiaque physiologique. L'invention permet de pallier ce problème en déterminant un profil de variation physiologique. La mise en oeuvre de l'invention est faite par un logiciel, exécuté par le micro-processeur du stimulateur cardiaque, mais pourrait aussi être réalisée par une électronique adaptée.

D'après la Figure 1c ainsi que la littérature médicale, l'évolution de la fréquence cardiaque d'un sujet soumis à un effort de niveau de puissance constant (comme une marche) est très bien corrélée avec une formulation mathématique de la forme

$$Fc(t) = A \{1-B.exp(-t/Tau)\} + C$$

dans laquelle F(c)t est la fréquence de stimulation, t est le temps, A, B, C et Tau sont des constantes, susceptibles d'être réglables ou programmables.

Selon l'invention, cette formule est choisie telle que :

Fc(t)=(Fdépart-Fbut)*exp(-t/Tau_mont)+Fbut , à la croissance
Fc(t)=(Fdépart-Fbut)*exp(-t/Tau_desc)+Fbut , à la décroissance
où Fbut est la fréquence qui doit être atteinte et qui est donnée par le paramètre : Fbut=Fcapt ; Fdépart peut être la fréquence de repos ou la fréquence maximale de stimulation ou la fréquence courante ; Tau_mont est un paramètre qui permet de contrôler la rapidité de la montée et Tau_desc un paramètre qui permet de contrôler la rapidité de la descente : ces deux paramètres sont déterminés en fonction du choix du médecin pour son patient. Ces deux équations sont identiques, ce qui permet un traitement homogène des profils de croissance et de décroissance de la fréquence cardiaque stimulée.

En pratique le stimulateur contrôle des intervalles d'échappement et travaille donc en période ( Tc=1/Fc ). A chaque temps de calcul on s'intéresse à la variation dTc qu'il faut appliquer à l'intervalle d'échappement cardiaque en cours pour calculer la durée de l'intervalle suivant. On ne s'intéresse donc qu'aux différentielles de ces équations (calculées en période) :

*    pour la croissance de la fréquence cardiaque :

```
dTc(t)    -F'c(t)
------=---------=-Tc²(t)*[-1/Tau_mont*(Fdépart-Fbut)exp(-t/Tau_mont)]
  dt      Fc²(t)
               = 1/Tau_mont*Tc²(t)*[Fc(t)-Fbut] < 0        (1)
```

*    pour la décroissance de la fréquence cardiaque

```
dTc(t)
------ =   -Tc²(t)*[-1/Tau_desc*(Fdépart-Fbut)exp(-t/Tau_desc)]
   dt
          = 1/Tau_desc*Tc²(t)*[Fc(t)-Fbut] > 0        (2)
```

Les intervalles de temps dt où ces calculs sont réalisés représentent au minimum un intervalle d'échappement cardiaque : dt=Tc(t) . Les relations (1) et (2) deviennent dans ce cas :

$$dTc(t) = 1/Tau\_mont*Tc^3(t)*[Fc(t)-Fbut] < 0 \qquad\qquad (3)$$

$$dTc(t) = 1/Tau\_desc*Tc^3(t)*[Fc(t)-Fbut] > 0 \qquad (4)$$

Il est plus simple d'avoir toujours dTc>0 et de l'ôter à l'intervalle d'échappement courant pour la croissance du rythme, et de l'ajouter pour la décroissance ; les relations (3) et (4) deviennent :

$$dTc(t) = 1/Tau\_mont*Tc^3(t)*[Fbut-Fc(t)] > 0 \qquad (5)$$

$$dTc(t) = 1/Tau\_desc*Tc^3(t)*[Fc(t)-Fbut] > 0 \qquad (6)$$

avec dTc, Tau, Tc exprimés en secondes et Fbut, Fc en Hertz.

Pour l'application décrite, les intervalles d'échappement sont exprimés en unités de 15.625ms et les fréquences en $mn^{-1}$ ; les relations (5) et (6) deviennent :

$$dTc(t) = Arrondi\{1/Tau\_mont*Tc^3(t)*[Fbut-Fc(t)]/3840\} > 0 \qquad (7)$$

$$dTc(t)\ Arrondi\{1/Tau\_desc*Tc^3(t)*[Fc(t)-Fbut]/3840\} > 0 \qquad (8)$$

Dans l'application décrite la plus petite valeur possible pour dTc est 15.625ms (1 unité) ; entre deux cycles cardiaques consécutifs la bonne description du profil, à l'approche de la consigne Fbut, nécessite l'application de valeurs inférieures à 15.625ms. Ce problème est résolu en n'appliquant pas dTc à chaque cycle cardiaque mais un cycle sur 2 ou sur 3, ou sur 4, etc, en fonction d'un coefficient multiplicateur MULT qui s'incrémente chaque fois que dTc vaut 0 (0 unité).

L'introduction de ce coefficient doit être comprise comme une multiplication de l'intervalle de calcul dt des équations (1) et (2). Dès lors le passage aux équations (3) et (4) se fait en prenant dt=MULT*Tc(t) . Tant que le calcul de dTc(t) donne des valeurs non nulles, MULT=1 ; et dès que dTc(t) vaut zéro le multiplicateur MULT est incrémenté à chaque intervalle d'échappement jusqu'à obtenir une valeur non nulle de dTc ; dès lors le multiplicateur MULT est remis à 1 pour les cycles suivants. Dès que la consigne Fbut est atteinte, le multiplicateur MULT est remis à 1. Les relations (7) et (8) deviennent :

$$dTc(t) = Arrondi\{MULT/Tau\_mont*Tc^3(t)*[Fbut-Fc(t)]/3840\} > 0 \qquad (9)$$

$$dTc(t) = Arrondi\{MULT/Tau\_desc*Tc^3(t)*[Fc(t)-Fbut]/3840\} > 0 \qquad (10)$$

Afin de prévenir un écart trop important de la fréquence cardiaque entre deux cycles consécutifs, la variation autorisée de la fréquence cardiaque d'un cycle au suivant est limitée à une valeur maximale (valeur butée). Le multiplicateur MULT est remis à 1 si la limitation est appliquée. Cette valeur butée est de préférence égale à 6 $mn^{-1}$.

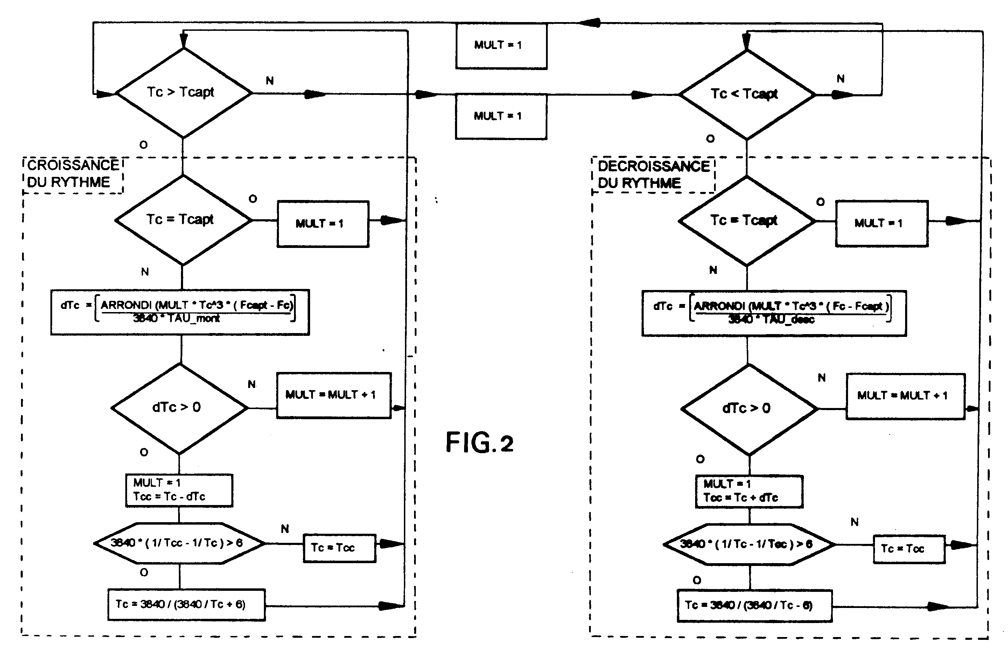

La figure 2 présente un organigramme de contrôle du profil de fréquence cardiaque exploitant les équations (9) et 10). Cet organigramme gère la croissance et la décroissance du rythme et calcule l'intervalle d'échappement suivant en fonction de celui du cycle en cours par :

* en croissance : Tc(t+1)=Tc(t)-dTc(t)  avec dTc(t) donné par (9)
* en décroissance : Tc(t+1)=Tc(t)+dTc(t)  avec dTc(t) donné par (10).

Le symbole Tcc désigne la période cardiaque calculée.

Dans l'application, la consigne Fbut est calculée tous les 1.56s indépendamment du calcul des intervalles d'échappement. Elle est utilisée lors du calcul de l'intervalle d'échappement à venir. Cette consigne peut varier d'un intervalle d'échappement à l'autre sans provoquer de discontinuité dans le calcul de la fréquence cardiaque asservie car le deux équations homogènes de la croissance et de la décroissance assurent la continuité mathématique.

Ce principe de contrôle de la fréquence cardiaque a permis d'obtenir des profils de fréquence cardiaque calculée (Figure 1b) similaires aux profils physiologiques relevés lors d'efforts de marche ou de course chez des sujets sans pathologie cardiaque (Figure 1c).

Selon l'invention, la fonction mathématique utilisée a pour objet de faire suivre à la fréquence de stimulation l'évolution du rythme cardiaque physiologique.

Dans l'exemple décrit ci-dessus, la fonction mathématique est du type

$$Fc(t) = A* (1 - B* exp. (-t/Tau)) + C$$

Cette fonction peut être remplacée par d'autres fonctions qui représentent une approximation de l'évolution du rythme cardiaque physiologique.

A titre d'exemples, sont à retenir dans le cadre de l'invention des fonctions mathématiques telles que :

$$Fc(t) = K + K' * t/Tau * [1 - t/ (2 Tau)]$$

ou $Fc(t) = K - (Tau/t)$ ,
dans lesquelles K ET K' sont des constantes,
qui sont des fonctions non-linéaires, et qui dépendent du temps et non du paramètre d'activité.

## Revendications

1.   Stimulateur cardiaque implantable à fréquence asservie à au moins un paramètre représentatif de l'activité physique du porteur, dans lequel l'évolution de la fréquence de stimulation suit, de façon à reproduire l'évolution du rythme cardiaque physiologique, une fonction mathématique Fc(t), caractérisé en ce que :

$$Fc(t)=A*(1-B*exp(-t/Tau))+C,$$

où

Fc(t) est la fréquence cardiaque délivrée par le stimulateur au cours du temps,

t le paramètre de temps et A, B, C, Tau des constantes.

2.   Stimulateur selon la revendication 1, caractérisé en ce que ladite fonction mathématique est approchée par un développement limité.

3.   Stimulateur selon la revendication 2, caractérisé en ce que la fonction mathématique est :

$$Fc(t) = K - (Tau/t).$$

4.   Stimulateur selon la revendication 1, caractérisé en ce que ladite fonction mathématique est :

-   $Fc(t)=(Fdépart-Fbut)exp(-t/Tau\_mont)+Fbut$ , pour contrôler la croissance du rythme stimulé,
-   $Fc(t)=(Fdépart-Fbut)exp(-t/Tau\_desc)+Fbut$ , pour contrôler la décroissance du rythme stimulé, où
-   Fdépart est la fréquence cardiaque à partir de laquelle se produit l'accélération ou la décélération du rythme stimulé,
-   Fbut est la fréquence cardiaque qui doit être atteinte et qui est calculée à partir de la valeur du ou des paramètres d'asservissement dudit stimulateur,
-   Tau_mont est un paramètre de réglage de la croissance du rythme stimulé depuis Fdépart jusqu'à Fbut,
-   Tau_desc est un paramètre de réglage de la décroissance du rythme stimulé depuis Fdépart jusqu'à Fbut.

5.   Stimulateur selon la revendication 4, caractérisé en ce que ladite fonction mathématique est transformée pour s'appliquer à des intervalles d'échappement cardiaque et autoriser le calcul de l'intervalle d'échappement suivant en ajoutant à l'intervalle d'échappement courant la variation :

-   $dTc(t)=Tc^{3}(t)*[Fc(t)-Fbut]/Tau$ , où
-   $Tc(t)=1/Fc(t)$
-   $Tau=Tau\_mont$ , pour la croissance du rythme,
-   $Tau=Tau\_desc$ , pour la décroissance du rythme.

6.   Stimulateur selon la revendication 5, caractérisé en ce que la variation dTc(t) n'est pas systématiquement ajoutée à chaque intervalle d'échappement mais à un intervalle d'échappement sur un nombre variable (MULT) d'intervalles d'échappement.

7. Stimulateur selon la revendication 6, caractérisé en ce que pour appliquer ladite variation dTc(t) à un intervalle d'échappement sur un nombre variable (MULT) d'intervalles d'échappement, ladite variation est calculée de la façon suivante :

- dTc(t)=Arrondi(MULT*Tc$^3$(t)*[Fc(t)-Fbut]/Tau) , où le facteur MULT est obtenu par un algorithme.

8. Stimulateur selon la revendication 7, caractérisé en ce que le facteur MULT :

- est incrémenté quand ladite variation appliquée à l'intervalle d'échappement précédent est nulle,
- est remis à 1 dès que ladite variation est non nulle,
- est remis à 1 si Fc=Fbut .

9. Stimulateur selon la revendication 8, caractérisé en ce que la variation autorisée de la fréquence cardiaque d'un cycle au suivant est limitée à une valeur maximale, de préférence égale à 6 min$^{-1}$.

**Claims**

1. An implantable cardiac pacemaker having a frequency controlled by at least one parameter representative of the physical activity of the wearer, wherein the change of the stimulation frequency follows, in a manner to reproduce the change of the physiological cardiac rhythm, a mathematical function Fc(t), characterised in that:

$$Fc(t) = A * (1 - B * e^{-t/Tau}) + C,$$

where

Fc(t) is the cardiac frequency delivered by the pacemaker as a function of time,
t is the parameter of time, and A, B, C, and Tau are constants.

2. The pacemaker as claimed in claim 1, characterised in that said mathematical function is approached by a limited expansion.

3. The pacemaker as claimed in claim 2, characterised in that the mathematical function is :

$$Fc(t)=K - (Tau/t)$$

4. The pacemaker as claimed in claim 1, characterised in that said mathematical function is :

- Fc(t) = (Fstart - Ftarget) e$^{-t/Tau\_inc}$ + Ftarget , to control the increase of the stimulation frequency, and
- Fc(t) = (Fstart - Ftarget) e$^{-t/Tau\_dec}$ + Ftarget , to control the decrease of the stimulated rhythm, where:
- Fstart is the cardiac frequency from which the increase or the decrease of the stimulated rhythm begins,
- Ftarget is the cardiac frequency that has to be reached and which is calculated on the basis of the value of one or more control parameters of the pacemaker,
- Tau_inc is a control parameter of the increase of the stimulated rhythm from Fstart to Ftarget,
- Tau_dec is a control parameter of the decrease of the stimulated rhythm from Fstart to Ftarget.

5. The pacemaker as claimed in claim 4, characterised in that said mathematical function is transformed to be applied at cardiac expulsion intervals and to authorise the calculation of the next expulsion interval by adding the following variation to the current expulsion interval:

$$dTc(t) =Tc^3(t) * [Fc(t) - Ftarget] / Tau, \text{ where}$$

$$Tc(t) = 1/Fc(t);$$

Tau=Tau_inc , for an increasing frequency,

Tau=Tau_dec , for a decreasing frequency.

6. The pacemaker as claimed in claim 5, characterised in that the variation dTc(t) is not systematically added to each expulsion interval but to an expulsion interval out of a variable number (MULT) of expulsion intervals.

7. The pacemaker as claimed in claim 6, characterised in that in order to apply said variation dTc(t) on an expulsion interval out of a variable number (MULT) of expulsion intervals, said variation is calculated as follows :

- $dTc(t) = Rounded (MULT * Tc^3(t) * [ Fc(t) - Ftarget]/Tau)$ , where the factor MULT is obtained by an algorithm.

8. The pacemaker as claimed in claim 7, characterised in that the factor MULT:

- is incremented when said variation applied on the preceding expulsion interval is zero,
- is reset to 1 as soon as the variation is non zero,
- is reset to 1 if Fc=Ftarget .

9. The pacemaker as claimed in claim 8, characterised in that the authorised variation of the cardiac frequency from one cycle to the following is limited to a maximal value, preferably equal to $6min^{-1}$.

**Patentansprüche**

1. Implantierbarer Herzschrittmacher mit einer Frequenz, die durch zumindest einen die physische Aktivität einer Trägerperson darstellenden Parameter geregelt wird, wobei die Entwicklung einer Stimulationsfrequenz einer mathematischen Funktion Fc(t) zur Reproduktion einer Entwicklung des physiologischen Herzrhythmus nachfolgt, dadurch gekennzeichnet, dass

$$Fc(t)=A*(1-B*exp(-t/Tau))+C$$

ist,

wobei

Fc(t) den zeitlichen Verlauf der durch den Herzschrittmacher abgegebene Herzfrequenz darstellt,
t ein Zeitparameter ist und A, B, C sowie Tau Konstanten sind.

2. Herzschrittmacher nach Anspruch 1, dadurch gekennzeichnet, dass die mathematische Funktion durch eine begrenzte Reihenentwicklung angenähert wird.

3. Herzschrittmacher nach Anspruch 2, dadurch gekennzeichnet, dass die mathematische Funktion

$$Fc(t)=K-(Tau/t)$$

ist.

4. Herzschrittmacher nach Anspruch 1, dadurch gekennzeichnet, dass die mathematische Funktion

- $Fc(t)=(Fstart-Fziel)exp(-t/Tau\_auf)+Fziel$ ist, damit die Zunahme des Stimulationsrhythmus geregelt wird,
- $Fc(t)=(Fstart-Fziel)exp(t/Tau\_ab)+Fziel$ ist, damit die Abnahme des Stimulationsrhythmus geregelt wird, wobei
- Fstart die Herzfrequenz ist, ausgehend von der die Beschleunigung oder die Verzögerung des Stimulationsrhythmus entsteht,
- Fziel die Herzfrequenz ist, die erreicht werden soll und die ausgehend von dem Wert des Parameters oder der Parameter zur Regelung des Herzschrittmachers berechnet wird,
- Tau_auf ein Parameter zur Regelung der Zunahme des Stimulationsrhythmus von Fstart zu Fziel ist und
- Tau_ab ein Parameter zur Regelung der Abnahme des Stimulationsrhythmus von Fstart zu Fziel ist.

5. Herzschrittmacher nach Anspruch 4, dadurch gekennzeichnet, dass die mathematische Funktion umgewandelt wird, damit sie für Herzausstoßintervalle angepasst ist und eine Berechnung des nachfolgenden Herzausstoßintervalls ermöglicht, indem dem derzeitige Herzausstoßintervall eine Variation hinzugefügt wird:

- dTc(t)=Tc$^3$(t)*[Fc(t)-Fziel]/Tau , wobei
- Tc(t)=1/Fc(t)  ist,
- Tau=Tau_auf  ist für die Zunahme des Rhythmus und
- Tau=Tau_ab  ist für die Abnahme des Rhythmus.

6. Herzschrittmacher nach Anspruch 5, dadurch gekennzeichnet, dass die Variation dTc(t) nicht systematisch zu jedem Ausstoßintervall hinzugefügt wird, sondern zu einem Ausstoßintervall aus einer variablen Anzahl (MULT) von Aus stoßintervallen.

7. Herzschrittmacher nach Anspruch 6, dadurch gekennzeichnet, dass zur Anwendung der Variation dTc(t) bei einem Ausstoßintervall aus einer variablen Anzahl (MULT) von Ausstoßintervallen die Variation berechnet wird nach

- dTc(t)=gerundet(MULT*Tc$^3$(t)*[Fc(t)-Fziel]/Tau ,
  wobei der Faktor MULT aus einem Algorithmus erhalten wird.

8. Herzschrittmacher nach Anspruch 7, dadurch gekennzeichnet, dass der Faktor MULT

- erhöht wird, wenn die dem vorherigen Ausstoßintervall zugeführte Variation Null ist,
- wieder auf 1 gesetzt wird, sobald die Variation nicht Null ist,
- wieder auf 1 gesetzt wird, wenn Fc=Fziel  ist.

9. Herzschrittmacher nach Anspruch 8, dadurch gekennzeichnet, dass die gestattete Variation der Herzfrequenz eines nachfolgenden Zyklus auf einen maximalen Wert begrenzt ist, vorzugsweise auf 6 min$^{-1}$.

## FIG.1a

Paramètre = f(t)

## FIG.1b

Fc_calculée = f(t)

## FIG.1c

Fc_mesurée = f(t)

Temps (mn:sec)

FIG.2